# EUROPEAN PATENT SPECIFICATION

(11) **EP 0 835 864 B1**
(45) Date of publication and mention of the grant of the patent: **13.02.2002**
(21) Application number: 97402385.5
(22) Date of filing: 10.10.1997
(51) Int. Cl.: C07C 231/02

(54) **A method of preparing primary amide derivatives**
Verfahren zur Herstellung von primären Amid-Derivaten
Procédé de préparation de dérivés des amides primaires

(30) Priority: 11.10.1996 JP 28735096
(43) Date of publication of application: 15.04.1998
(73) Proprietor: Takasago International Corporation, Tokyo 108 (JP)
(72) Inventor: Sakurai, Kazutoshi Takasago International Corp., Kanagawa-ken (JP); Ishida, Kenya Takasago International Corp., Kanagawa-ken (JP); Ogura, Miharu Takasago International Corp., Kanagawa-ken (JP)
(74) Representative: Gillard, Marie-Louise

(56) References cited:
- EP-A- 0 373 038
- EP-A- 0 482 860
- EP-A- 0 500 437
- K. MORI ET AL : "Stereochemistry of aplidiasphingosine as proposed by the asymmetric synthesis and 13C-NMR study of sphingosine relatives" TETRAHEDRON LETTERS, vol. 22, no. 44, 1981, pages 4433-4436, XP002082490 OXFORD GB

## Description

The present invention relates to novel methods of synthesising primary amide derivatives. More particularly, the present invention relates to methods of synthesising a primary amide derivative and an optically active form thereof. The methods consist of reacting an aminodiol having a primary and a secondary hydroxyl group with a fatty acid alkyl ester in an alcohol-containing solvent, so as to selectively acylate the amino group of the aminodiol.

In conventional methods for synthesising amide derivatives from amino alcohol derivatives, the amino alcohol derivatives are acylated with acid anhydrides, acyl halides, fatty acid esters, or fatty acids in the absence of a catalyst, or in the presence of a basic catalyst such as a sodium alkoxide, triethylamine, or pyridine. However, these methods have a disadvantage in that a stoichiometrically excess amount of base has to be added to the raw aminodiol material, leading to the formation of a salt that has to be removed after reaction by an additional process.

On the other hand, a method for synthesising a primary amide compound from a primary amine compound and a fatty acid ester is disclosed by F.J. Edmund et al. in J. Am. Oil Chem. Soc., 38, 600 (1961). In this method, solvents and reagents have to be distilled and dried prior to use. When this method is applied to an aminodiol compound having hydroxyl groups of both a primary and a secondary alcohol, the primary alcohol hydroxyl group is also acylated. This decreases yield of the sought-after product. Furthermore, to remove ester-based by-products, hydrolysis must be carried out. This hydrolysis forms a large amount of fatty acid salts. To remove these salts, a cumbersome additional procedure must be employed.

Japanese Unexamined Patent Publication No. 63-216,852 discloses N-acylation of secondary amines having the hydroxyl groups of both a primary and a secondary alcohol with methyl esters of fatty acids. This N-acylation does not use any solvent and is effected in the presence of a basic catalyst. The use of a catalytic amount of a polyether compound provides a more satisfactory result.

Japanese Unexamined Patent Publication No. 4-282,304 discloses a method of synthesising a secondary amide compound by N-acylation. In this method, a secondary amine having primary and secondary hydroxyl groups is reacted with methyl esters of 2-hydroxy fatty acids without using any solvent.

However, according to supplementary experiments conducted by the present inventors, the sought-after primary amide product cannot form effectively an aminodiol compound having primary and secondary hydroxyl groups through the above-mentioned conventional amidation process, regardless of whether the reaction is carried out in the absence of a solvent or with a small amount of a polyether compound. More particularly, the primary amide derivative formed during the reaction crystallises easily and has a high melting point. Consequently, the crystallised derivative prevents efficient stirring and leads to an incomplete reaction.

Heating the reaction in order to complete it leads to a side reaction due to esterification of the primary alcohol. Furthermore, since the amide product has completely crystallised after cooling the reaction mixture, removal of the product from the reactor is difficult industrially.

As set forth above, when aminodiols are N-acylated without solvent, the resulting amide derivatives crystallise and solidify as the reaction proceeds. Consequently, it is difficult to remove the products from the reactor. When using a solvent such as a polyether compound, crystallisation still occurs during the reaction and cooling, so that another solvent is required to remove the product from the reactor.

It is therefore an object of the present invention to provide a more efficient method of selectively N-acylating the amino group of an aminodiol having primary and secondary hydroxyl groups, wherein the product is not crystallised during the reaction and wherein an additional cumbersome procedure is not required for extracting and purifying the sought-after product after reaction.

The present inventors investigated a method of synthesising a primary amide derivative by selectively N-acylating the amino group of an aminodiol having primary and secondary hydroxyl groups, using a solvent. The inventors discovered that when an alcohol, that does not crystallise at room temperature and whose boiling point is relatively high, is used as a solvent, the sought-after product is efficiently produced.

More particularly, the present invention provides a method of synthesising a primary amide derivative represented by the following general formula (3): 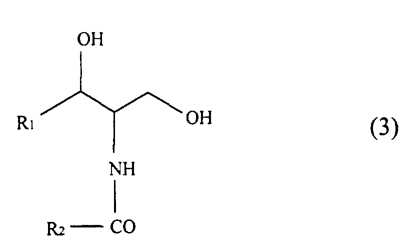 wherein R₁ represents a saturated linear hydrocarbon group having 11 to 19 carbon atoms, and R₂ represents a saturated or unsaturated linear hydrocarbon group having 9 to 19 carbon atoms which may or may not have a hydroxyl group in the 1-position, said method comprising the step of :
- reacting an aminodiol represented by the following general formula (1), 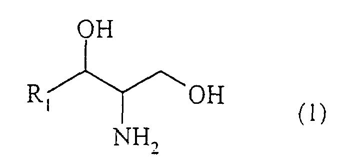 wherein R₁ is the same as R₁ in general formula (3) with a fatty acid alkyl ester represented by the following general formula (2):

   R₂―COO―R₃ (2)

   wherein R₂ is the same as R₂ in general formula (3) and R₃ represents a lower alkyl group having 1 to 6 carbon atoms, in a reaction system comprising an alcohol-based solvent and a basic catalyst, said reaction system forming lower alcohols (R₃OH) as by-product; and
- removing the lower alcohols, formed during the reaction, from the reaction system; and
- recovering the solution from the reaction system and cooling the residue to a temperature comprised between -10°C and room temperature to precipitate crude crystals; and
- collecting and recrystallising the crude crystals in methanol, ethanol or acetonitrile.

In another embodiment, the present invention provides a method of synthesising an optically active form of primary amide derivatives represented by the following general formula (5): 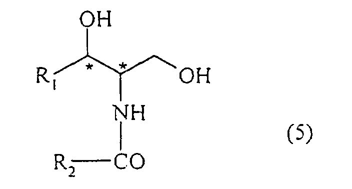 wherein R₁ represents a saturated linear hydrocarbon group having 11 to 19 carbon atoms, R₂ represents a saturated or unsaturated linear hydrocarbon group having 9 to 19 carbon atoms which may or may not have a hydroxyl group in the 1-position, and the symbol * representing an asymmetric carbon atom, said method comprising the steps of:
- reacting an optically active aminodiol represented by general formula (4): 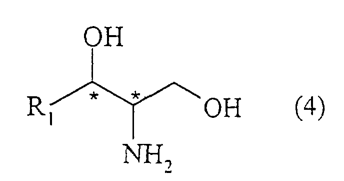 wherein R₁ and the symbol * are the same as R₁ and * in general formula (5), with a fatty acid alkyl ester represented by the following general formula (2):

   R₂―COO―R₃ (2)

   wherein R₂ is the same as R₂ in general formula (5) and R₃ represents a lower alkyl group having 1 to 6 carbon atoms, in a reaction system comprising an alcohol-based solvent and a basic catalyst; said reaction step forming lower alcohols (R₃OH) as by-product; and
- removing the lower alcohols, formed during the reaction, from the reaction system; and
- recovering the solution from the reaction system and cooling the residue to a temperature comprised between -10°C and room temperature to precipitate crude crystals; and
- collecting and recrystallising the crude crystals in methanol, ethanol or acetonitrile.

In accordance with a preferred embodiment of the present invention the removing step comprises removing the lower alcohols, formed during the reaction, from the reaction system.

The method of the present invention is preferably used for preparing a (2S,3R)-2-acylamino-1,3-diol as an optically active primary amide derivative represented by the general formula (5).

In the method of the present invention, when an alcohol-based solvent having a low solubility in water is used, a primary amide derivative can be produced in high yield by reacting an aminodiol having a primary and a secondary hydroxyl group with a fatty acid alkyl ester in the presence of a basic catalyst. The lower alcohols crystallisation still occurs during the reaction and cooling, so that another solvent is required to remove the product from the reactor.

It is therefore an object of the present invention to provide a more efficient method of selectively N-acylating the amino group of an aminodiol having primary and secondary hydroxyl groups, wherein the product is not crystallised during the reaction and wherein an additional cumbersome procedure is not required for extracting and purifying the sought-after product after reaction.

The present inventors investigated a method of synthesising a primary amide derivative by selectively N-acylating the amino group of an aminodiol having primary and secondary hydroxyl groups, using a solvent. The inventors discovered that when an alcohol, that does not crystallise at room temperature and whose boiling point is relatively high, is used as a solvent, the sought-after product is efficiently produced.

More particularly, the present invention provides a method of synthesising a primary amide derivative represented by the following general formula (3): 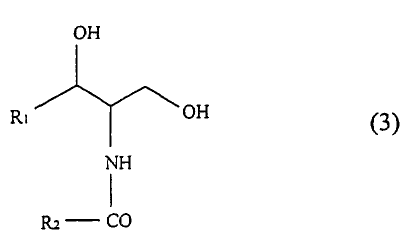 wherein R₁ represents a saturated linear hydrocarbon group having 11 to 19 carbon atoms, and R₂ represents a saturated or unsaturated linear hydrocarbon group having 9 to 19 carbon atoms which may or may not have a hydroxyl group in the 1-position, said method comprising the steps of :
- reacting an aminodiol represented by the following general formula (1),
   (compound D) is then prepared by deacetylating the ester group through reduction. 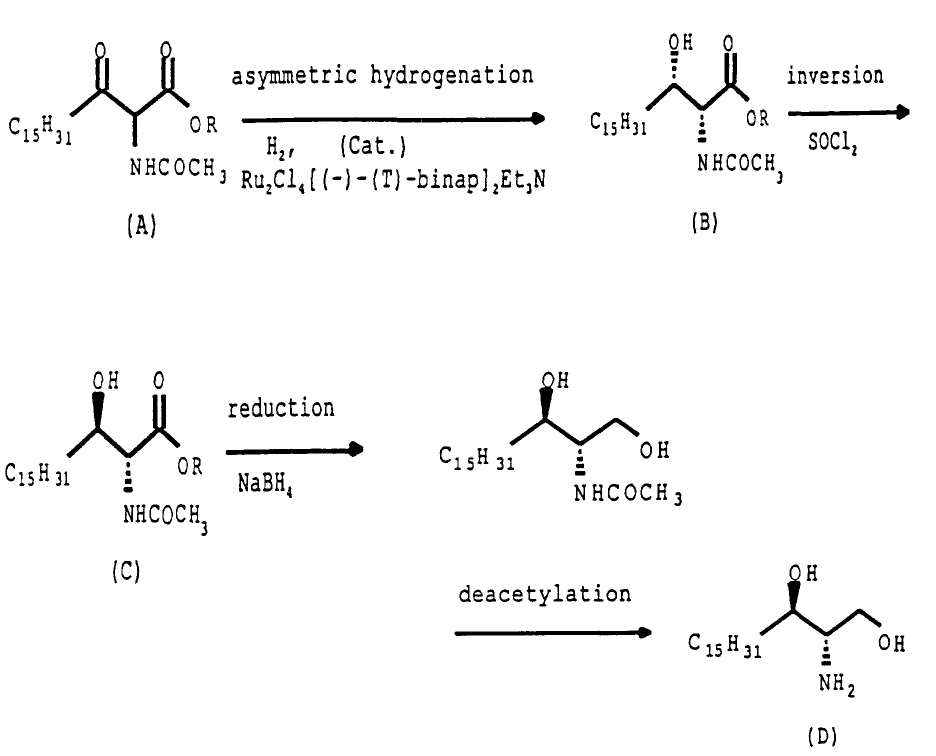
R in compounds A, B and C represents a lower alkyl group, and (T)-binap is 2,2'-bis(di-p-tolylphosphino-1,1'-binaphthyl)

If the hydroxyl group is not inverted, a (2S,3S)-isomer can be synthesised. In a same manner, compounds having different steric configurations can be prepared with various catalytic ligands. For example, a (2R,3R)-isomer and a (2R,3S)-isomer may be prepared by using a (+)-phosphine complex.

Examples of the fatty acid alkyl esters represented by the general formula (2):

R₂-COO-R₃ (2)

wherein R₂ represents a saturated or unsaturated linear aliphatic hydrocarbon group having 9 to 19 carbon atoms which may or may not have a hydroxyl group in the 1-position and R₃ represents a lower alkyl group having 1 to 6 carbon atoms, include methyl and ethyl esters of fatty acids, e.g., methyl decanoate, ethyl decanoate, methyl undecanoate, ethyl undecanoate, methyl dodecanoate, ethyl dodecanoate, methyl tridecanoate, ethyl tridecanoate, methyl tetradecanoate, ethyl tetradecanoate, methyl pentadecanoate, ethyl pentadecanoate, methyl hexadecanoate, ethyl hexadecanoate, methyl cis-9-hexadecenoate, ethyl cis-9-hexadecenoate, methyl heptadecanoate, ethyl heptadecanoate, methyl octadecanoate, ethyl octadecanoate, methyl cis-9-octadecenoate, ethyl cis-9-octadecenoate, methyl cis, cis-9,12-octadecadienoate, ethyl cis, cis-9,12-octadecadienoate, methyl nonadecanoate, ethyl nonadecanoate, methyl eicosanoate and ethyl eicosanoate.

Examples of the fatty acid esters having a hydroxyl group at the 1-position of R2, i.e. alkyl esters of 2-hydroxy fatty acids, include methyl 2-hydroxydecanoate, ethyl 2-hydroxydecanoate, methyl 2-hydroxyundecanoate, ethyl 2-hydroxyundecanoate, methyl 2-hydroxydodecanoate, ethyl 2-hydroxydodecanoate, methyl 2-hydroxytridecanoate, ethyl 2-hydroxytridecanoate, methyl 2-hydroxytetradecanoate, ethyl 2-hydroxytetradecanoate, methyl 2-hydroxypentadecanoate, ethyl 2-hydroxypentadecanoate, methyl 2-hydroxyhexadecanoate, ethyl 2-hydroxyhexadecanoate, methyl 2-hydroxyheptadecanoate, ethyl 2-hydroxyheptadecanoate, methyl 2-hydroxyoctadecanoate, ethyl 2-hydroxyoctadecanoate, methyl 2-hydroxynonadecanoate, ethyl 2-hydroxynonadecanoate, methyl 2-hydroxyeicosanoate and ethyl 2-hydroxyeicosanoate.

Among the lower alkyl groups of the esters, there are included straight and branched lower alkyl groups having 1 to 4 carbon atoms, preferably methyl and ethyl groups.

In the method of the present invention, the lower alcohols (R₃OH) having a boiling point lower than the alcohol-based solvent are removed from the reaction system.

The fatty acid alkyl ester represented by general formula (2) is used in an amount ranging from 1 to 1.5 moles, preferably from 1.1 to 1.5 moles, per mole of the aminodiol represented by general formula (1) in the reaction of the present invention.

Examples of alcohol-based solvents used in the reaction system include alcohols having 3 to 8 carbon atoms, e.g., n-propyl alcohol, isopropyl alcohol, n-butyl alcohol, isobutyl alcohol, amyl alcohol, hexyl alcohol, heptyl alcohol and octyl alcohol. These alcohols may be used alone or in combination. Among them, n-butyl alcohol is preferred because its handling and removal from the reaction solution are easy.

The alcohol solvent may be used in an amount ranging from 5 to 10 fold by weight, preferably from 7 to 8 fold by weight, relative to the amount of the aminodiol represented by the general formula (1). If the amount of the alcohol solvent is too low, a primary alcohol hydroxyl group is esterified to give by-products. Since the sought-after product and these by-products are both highly crystalline, they may not be separated by recrystallisation. It is also difficult to remove the by-products from the reaction mixture by distillation.

Examples of basic catalyst used in the present invention include sodium hydroxide, potassium hydroxide and sodium alkoxides. The basic catalyst may be used in an amount ranging from 0.01 to 0.2 mole per mole of the fatty acid alkyl ester represented by the general formula (2).

In the present invention, the fatty acid alkyl ester represented by the general formula (2) is added dropwise to the reaction solution containing an aminodiol represented by the general formula (1) or (4) and a basic catalyst, over a period ranging from 15 to 30 minutes. Alternatively, the fatty acid alkyl ester may be added at a time after the reaction solution was heated during the period of 30 minutes to 1 hour.

According to the present invention, the amino group of the aminodiol having both primary and secondary hydroxyl groups is selectively N-acylated. This N-acylation is effectively carried out by removing, from the reaction system, the lower alcohols such as methanol or ethanol which are formed during the acylation. The reaction may be effected under reduced pressure or in an inert gas atmosphere. For example, the reaction may be carried out under a reduced pressure ranging from 3.99 kPa to 13.33 kPa 30 mm Hg to 100 mm Hg) at a temperature in the range of 90 to 120°C for 1 to 2 hours. Alternatively, the reaction may be carried out under a stream of inert gas. For example, nitrogen gas may be fed through the reaction vessel at a rate of 20 ml/min, at a temperature of 90 to 120°C for approximately 1.5 hours.

After the reaction, the solution is removed from the reactor and the residue is cooled to a temperature comprised between -10°C and room temperature, in order to precipitate crude crystals of the sought-after primary amide derivative represented by general formula (3) or (5). The crude crystals are collected and recrystallised in methanol, ethanol or acetonitrile for the purpose of purification.

In the method according to the present invention, a primary amide derivative can be produced in high yield by reacting an aminodiol having primary and secondary hydroxyl groups with a fatty acid alkyl ester, in an alcohol-based solvent and in the presence of a basic catalyst. In this reaction, the lower alcohols formed during the reaction are removed from the reaction system. Furthermore, the reaction does not entail crystallisation, so that cumbersome post-operation, such as extraction or subsequent purification is avoided.

The resulting primary amide derivative is useful for enhancing the water retention capacity of the skin, and is also useful as a base agent for cosmetics and the like.

The following examples illustrate preferred embodiments of the present invention. However, the present invention should not be construed as being limited thereto. In the examples, the following analytical instruments and materials were used for identifying the reaction products.

### Analytical Instruments and Materials

* High Performance Liquid Chromatograph :
   Waters 510 (made by Waters, Ltd.) ;
   Detector : Waters 484 UV detector (made by Waters, Ltd.).
* Nuclear Magnetic Resonance Spectrometer :
   AM-400 (400 MHz: made by Bruker Inc.);
   Internal standard: tetramethylsilane;
      In the results, abbreviations 'br' and 'br s' signify 'broad' and 'broad singlet', respectively.
* Elemental Analyzer: CHN-2400 (made by Perkin Elmer, Ltd.).
* Mass Spectrometer: M80B (made by Hitachi, Ltd.).

### Example 1

### Synthesis of 2-octadecanoylaminohexadecane-1,3-diol

Into a 100 ml four-neck flask equipped with a stirrer, a dropping funnel and a thermometer, were placed 50 mg of commercially available 85% potassium hydroxide, 1.5 g (5 mmol) of 2-aminohexadecane-1,3-diol and 15 ml of butanol. The mixture was heated to 90 °C and stirred for 30 minutes. 2.24 g (7.5 mmol) of methyl stearate (methyl octadecanoate) in 5 ml of a butanol solution were then added dropwise thereto while feeding nitrogen into the reaction system. After stirring at 90°C for 2 hours, the reaction mixture was cooled and the resulting crude crystals were collected. The crude crystals were washed with methanol and dried, to give 2-octadecanoylaminohexadecane-1,3-diol. Yield was 87.2%.
Melting Point: 96.7 to 98.2 °C
¹H-NMR (CDCl₃ : CD₃OD = 10 : 1, δ) :
0.88 (6H, t, J=7.0 Hz),
1.27 (50H, br s), 1.54 (2H, m),
1.64 (2H, m), 2.23 (2H, t, J=7.6 Hz),
3.63 (1H, m),
3.68 (1H, dd, J=3.3, 11.5 Hz),
3.80 (1H, m),
3.83 (1H, dd, J=3.3, 11.5 Hz),
6.37 (1H, d, J=7.8 Hz)

MS : 540 (M⁺ + 1 )

| Elemental Analysis (as C₃₄H₆₉NO₃) : | | | |
|---|---|---|---|
| Theoretical (%) | C : 75.64, | H : 12.88, | N : 2.59 |
| Observed (%) | C : 75.60, | H : 12.78, | N : 2.66 |

### Example 2

### Synthesis of 2-hexadecanoylaminohexadecane-1,3-diol

Using the same procedure as in Example 1, 2-hexadecanoylaminohexadecane-1,3-diol was prepared from methyl palmitate (methyl hexadecanoate) and 2-aminohexadecane-1,3-diol. The yield was 89.3%.
Melting Point: 99.3 to 103.4 °C
¹H-NMR (CDCl₃: CD₃OD = 10 : 1, δ) :
0.88 (6H, t, J=6.9 Hz),
1.27 (46H, br s), 1.54 (2H, m),
1.64 (2H, m), 2.22 (2H, t, J=7.8 Hz),
3.62 (1H, m),
3.68 (1H, dd, J=3.4, 11.2 Hz),
3.79 (1H, m),
3.84 (1H, dd, J=3.4, 11.2 Hz),
6.37 (1H, d, J=7.7 Hz)

MS : 512 (M⁺ + 1)

| Elemental Analysis (as C₃₂H₆₅NO₃) : | | | |
|---|---|---|---|
| Theoretical (%) | C : 75.09, | H:12.80, | N : 2.74 |
| Observed (%) | C : 75.12, | H : 12.70, | N : 2.69 |

### Example 3

### Synthesis of 2-linoleoylaminohexadecane-1,3-diol

Using the same procedure as in Example 1, 2-linoleoylaminohexadecane-1,3-diol was prepared from methyl linoleate (methyl cis,cis-9,12-octadecadienoate) and 2-aminooctadecane-1,3-diol. The yield was 86.1%.
Melting Point : 92 to 99.5 °C
¹H-NMR (CDCl₃ : CD₃OD = 10 : 1, δ) :
0.88 (6H, t, J=7.0 Hz),
1.27 (48H, br s), 1.54 (2H, m),
1.64 (2H, m),
2.23 (2H, t, J=7.8 Hz),
3.76 (1H, dd, J=3.5, 11.3 Hz),
3.78 (1H, m),
4.01 (1H, dd, J=3.5, 11.3 Hz),
5.34 (4H, m),
6.37 (1H, d, J=7.8 Hz)

MS : 564 (M⁺ + 1)

| Elemental Analysis (as C₃₆H₆₉NO₃) : | | | |
|---|---|---|---|
| Theoretical (%) | C : 76.67, | H : 12.33, | N : 2.48 |
| Observed (%) | C : 76.74, | H : 12.38, | N : 2.49 |

### Example 4

### Synthesis of (2S,3R)-2-octadecanoylaminooctadecane-1,3-diol.

Into a 100 ml four-neck flask equipped with a stirrer, a dropping funnel and a thermometer, were placed 50 mg of commercially available 85% potassium hydroxide, 1.5 g (5 mmol) of (2S,3R)-2-aminooctadecane-1,3-diol and 15 ml of butanol. The mixture was heated to 90°C and stirred for 30 minutes. 2.24 g (7.5 mmol) of methyl stearate (methyl octadecanoate) in 5 ml of butanol solution were then dropwise added thereto while feeding nitrogen into the reaction system. After stirring at 90 °C for 2 hours, the reaction mixture was cooled and the resulting crude crystals were collected. The crude crystals were recrystallised from methanol and dried, to give 2.3 g of (2S,3R)-2-octadecanoylaminooctadecane-1,3-diol. Yield was 82.4%.
Melting Point : 105 to 106 °C
Optical Rotation: [α]$\frac{\text{25}}{\text{D}}$ +3.97°
(c = 0.13, CHCl₃ : CH₃OH = 10 : 1)
¹H-NMR (CDCl₃ : CD₃OD = 10 : 1, δ):
0.88 (6H, t, J=7.0 Hz),
1.27 (54H, br s), 1.54 (2H, m),
1.64 (2H, m), 2.23 (2H, t, J=7.8 Hz),
3.76 (1H, dd, J=3.3, 11.5 Hz),
3.78 (1H, m),
4.02 (1H, dd, J=3.3, 11.5 Hz),
6.38 (1H, d, J=7.8 Hz)

MS : 568 (M⁺ + 1)

| Elemental Analysis (as C₃₆H₇₃NO₃) : | |
|---|---|
| Theoretical (%) | C : 76.13, H : 12.95, N : 2.47 |
| Observed (%) | C : 76.04, H : 12.90, N : 2.51 |

### Example 5

### Synthesis of (2S,3R)-2-tetradecanoylaminooctadecane-1,3-diol

Using the same procedure as in example 4, (2S,3R)-2-tetradecanoylaminooctadecane-1,3-diol was prepared from methyl myristate (methyl tetradecanoate) and (2S,3R)-2-aminooctadecane-1,3-diol. The yield was 87.1%.
Melting Point : 101 to 103 °C
Optical Rotation : [α]$\frac{\text{25}}{\text{D}}$ +3.27°
(c = 0.122, CHCl₃: CH₃OH = 10 : 1)
¹H-NMR (CDCl₃ : CD₃OD = 10 : 1, δ) :
0.88 (6H, t, J=7.0 Hz),
1.27 (48H, br s), 1.54 (2H, m),
1.64 (2H, m),
2.23 (2H, dd, J=7.4, 7.8 Hz),
3.76 (1H, dd, J=3.5, 11.5 Hz),
3.78 (1H, m),
4.01 (1H, dd, J=3.5, 11.5 Hz),
6.37 (1H, d, J=7.8 Hz)

MS : 512 (M⁺ + 1)

| Elemental Analysis (as C₃₂H₆₅NO₃) : | |
|---|---|
| Theoretical (%) | C : 75.09, H : 12.80, N : 2.74 |
| Observed (%) | C : 75.15, H : 12.70, N : 2.69 |

### Example 6

### Synthesis of (2S,3R)-2-tetradecanoylaminooctadecane-1,3-diol

Using the same procedure as in Example 4, (2S,3R)-2-hexadecanoylaminooctadecane-1,3-diol was prepared from methyl palmitate (methyl hexadecanoate) and (2S,3R)-2-aminooctadecane-1,3-diol. The yield was 85.7%.
Melting Point : 105.5 to 106.7 °C
Optical Rotation: [α]$\frac{\text{25}}{\text{D}}$ +3.94°
(c = 0.12, CHCl₃: CH₃OH = 10 : 1)
¹H-NMR (CDCl₃ : CD₃OD = 10 : 1, δ):
0.88 (6H, t, J=7.0 Hz),
1.27 (52H, br s), 1.54 (2H, m),
1.64 (2H, m),
2.23 (2H, t, J=7.8 Hz),
3.76 (1H, dd, J=3.5, 11.5 Hz),
3.78 (1H, m),
4.01 (1H, dd, J=3.3, 11.5 Hz),
6.37 (1H, d, J=7.8 Hz)

MS : 540 (M⁺ + 1)

| Elemental Analysis (as C₃₄H₆₉NO₃) : | | | |
|---|---|---|---|
| Theoretical (%) | C : 76.64, | H : 12.88, | N : 2.59 |
| Observed (%) | C : 76.68, | H : 12.90, | N : 2.49 |

### Example 7

### Synthesis of (2S,3R)-2-oleoylaminooctadecane-1,3-diol

Using the same procedure as in Example 4, (2S,3R)-2-oleoylaminooctadecane-1,3-diol was prepared from methyl oleate (methyl cis-9-octadecenoate) and (2S,3R)-2-aminooctadecane-1,3-diol. The yield was 86.5%.
Melting Point: 96.5 to 99.5 °C
Optical Rotation : [α]$\frac{\text{25}}{\text{D}}$ +3.25°
   (c = 0.3, CHCl₃ : CH₃OH = 10 : 1)
¹H-NMR (CDCl₃ : CD₃OD = 10 : 1, δ) :
   0.88 (6H, t, J=7.0 Hz),
   1.27 (48H, br s), 1.54 (2H, m),
   1.64 (2H, m), 2.00 (4H,m),
   2.23 (2H, t, J=7.8 Hz),
   3.76 (1H, dd, J=3.3, 11.5 Hz),
   3.78 (1H, m),
   4.01 (1H, dd, J=3.5, 11.5 Hz),
   5.34 (2H, m),
   6.94 (1H, d, J=8.2 Hz)
MS : 566 (M⁺ + 1)

### Example 8

### Synthesis of (2S,3R,2'RS)-2-(2'-hydroxydecanoyl)aminoocta decane-1,3-diol

Using the same procedure as in Example 4, (2S,3R,2'RS)-2-(2'-hydroxydecanoyl) aminooctadecane-1,3-diol was prepared from methyl 2-hydroxydecanoate and (2S,3R)-2-aminooctadecane-1,3-diol. The yield was 90.5%.
Melting Point: 89 to 92 °C
¹H-NMR (CDCl₃ : CD₃OD = 10 : 1, δ) :
0.86 (3H, t, J=7.0 Hz),
0.88 (3H, t, J=7.0 Hz),
1.27 (38H, br s), 1.64 (2H, m),
2.30 (1H, dd, J=9.3, 15.0 Hz),
2.43 (1H, dd, J=2.7, 15.0 Hz),
2.50 (1H, d, J=6.9 Hz),
2.62 (1H, br), 3.44 (2H, br),
3.80 (1H,m),
4.01 (1H, dd, J=3.0, 9.3 Hz),
6.60 (1H, d, J=6.9 Hz)

MS : 471 (M⁺ + 1)

| Elemental Analysis (as C₂₈H₅₇NO₄) : | | | |
|---|---|---|---|
| Theoretical (%) | C : 71.28, | H : 12.18, | N : 2.97 |
| Observed (%) | C : 71.35, | H : 12.20, | N : 2.95 |

### Example 9

### Synthesis of (2S,3R,2'RS)-2-('2'-hydroxyhexadecanoyl)-aminooctadecane-1,3-diol

Using the same procedure as in Example 4, (2S,3R,2'RS)-2-(2'-hydroxyhexadecanoyl)aminooctadecane-1,3-diol was prepared from methyl 2-hydroxyhexadecanoate and (2S,3R)-2-aminooctadecane-1,3-diol. The yield was 90 %.
Melting Point : 112 to 122 °C
¹H-NMR (CDCl₃ : CD₃OD = 10 : 1, δ) :
0.86 (3H, t, J=7.0 Hz),
0.88 (3H, t, J=7.0 Hz),
1.27 (50H, br s), 1.64 (2H, m),
2.30 (1H, dd, J=9.3, 15.0 Hz),
2.43 (1H, dd, J=2.7, 15.0 Hz),
2.50 (1H, d, J=6.9 Hz),
2.62 (1H, br), 3.44 (2H, br),
3.80 (1H, m),
4.01 (1H, dd, J=3.0, 9.3 Hz),
6.60 (1H, d, J=6.9 Hz)

MS : 556 (M⁺ + 1)

| Elemental Analysis (as C₃₄H₆₉NO₄) : | | | |
|---|---|---|---|
| Theoretical (%) | C : 73.46, | H : 12.51, | N : 2.52 |
| Observed (%) | C : 73.35, | H : 12.40, | N : 2.49 |

In the following Comparative Examples, supplementary experiments were carried out following the method described in Japanese Unexamined Patent Publication No. 63-216,852.

### Comparative Example 1

### Synthesis of (2S,3R)-2-octadecanoylaminooctadecane-1,3-diol.

Into a 100 ml four-neck flask equipped with a stirrer, a dropping funnel and a thermometer, were placed 50 mg of commercially available 85% potassium hydroxide and 1.5 g (5 mmol) of (2S,3R)-2-aminooctadecane-1,3-diol. The mixture was heated to 90 °C and stirred for 30 minutes. While feeding nitrogen into the reaction system, 2.24 g (7.5 mmol) of methyl stearate (methyl octadecanoate) were added dropwise thereto. After a period of 30 minutes, crystals precipitated, thus preventing the stirrer from running. Ethanol was added into the reactor to dissolve the crystals. The resultant solution was then subjected to recrystallisation, to give 1.59 g of (2S,3R)-2-octadecanoylaminooctadecane-1,3-diol. Yield was 56%.

### Comparative Example 2

### Synthesis of (2S,3R)-2-octadecanoylaminooctadecane-1,3-diol.

Into a 100 ml four-neck flask equipped with a stirrer, a dropping funnel and a thermometer, were placed 50 mg of commercially available 85% potassium hydroxide and 1.5 g (5 mmol) of (2S,3R)-2-aminooctadecane-1,3-diol. The mixture was then heated to 90 °C and stirred for 30 minutes. While feeding nitrogen into the reaction system, 2.24 g (7.5 mmol) of methyl stearate (methyl octadecanoate) were added dropwise thereto. Stirring was interrupted after a period of 30 minutes due to the precipitation of a crystalline product ; the contents were therefore heated to 120 °C. The heated mixture was recovered without cooling and subjected to crystallisation. The product obtained was recrystallised with ethanol or methanol. This product (2.04 g) was a mixture of (2S,3R)-2-octadecanoylaminooctadecane-1,3-diol and (2S,3R)-3-hydroxy-2-octadecanoylamino-1-octadecanoyloxy octadecane, which were not separable by recrystallisation.

### Comparative Example 3

### Synthesis of (2S,3R)-2-octadecanoylaminooctadecane-1,3-diol

Into a 100 ml four-neck flask equipped with a stirrer, a dropping funnel and a thermometer, were placed 50 mg of commercially available 85% potassium hydroxide and 1.5 g (5 mmol) of (2S,3R)-2-aminooctadecane-1,3-diol. The mixture was heated to 90 °C and stirred for 30 minutes. While feeding nitrogen into the reaction system, 2.24 g (7.5 mmol) of methyl stearate (methyl octadecanoate) were added dropwise thereto. Stirring was hindered after a period of 30 minutes due to precipitation of a crystalline product ; the contents were therefore heated to 120°C and further reacted for 2 hours. The heated product was recovered without cooling and subjected to crystallisation. The product obtained was recrystallized with ethanol. This product (2.00 g) was a mixture of (2S,3R)-2-octadecanoylaminooctadecane-1,3-diol, (2S,3R)-3-hydroxy-2-octadecanoylamino-1-octadecanoyloxy octadecane and other unidentified compounds which could not be separated.

As set forth above, a solvent was not used in Comparative Example 1. Consequently, the reaction was incomplete due to crystalline precipitation and the yield was unsatisfactorily low. When the reaction conditions were modified so that the crystals did not precipitate (Comparative Examples 2 and 3), the resulting products were mixtures containing the sought-after product and other compounds. However, the desired products could not be isolated from the respective mixtures.

## Claims

1. A method of preparing a primary amide derivative represented by general formula (3): 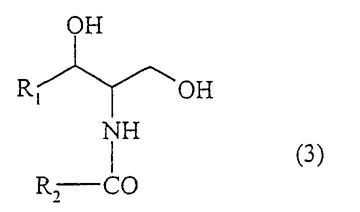 wherein R₁ represents a saturated linear hydrocarbon group having 11 to 19 carbon atoms, and R₂ represents a saturated or unsaturated linear hydrocarbon group having 9 to 19 carbon atoms which may or may not have a hydroxyl group in the 1-position, said method comprising the steps of:
- reacting an aminodiol represented by general formula (1): 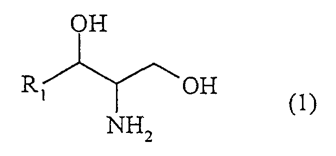
wherein R₁ is the same as R₁ in general formula (3), with a fatty acid alkyl ester represented by general formula (2):
R₂―COO―R₃ (2)
wherein R₂ is the same as R₂ in general formula (3) and R₃ represents a lower alkyl group having 1 to 6 carbon atoms, in a reaction system comprising an alcohol having 3 to 8 carbon atoms and a basic catalyst, said reaction system forming R₃OH as by-product; and
- removing R₃OH, formed during the reaction, from the reaction system;
- recovering the solution from the reaction system and cooling the residue to a temperature comprised between -10°C and room temperature to precipitate crude crystals; and
- collecting and recrystallising the crude crystals in methanol, ethanol or acetonitrile.

2. A method of preparing an optically active primary amide derivative represented by general formula (5): 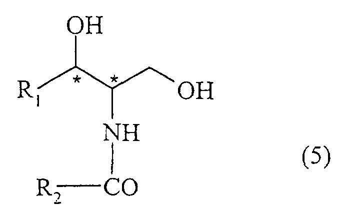 wherein R₁ represents a saturated linear hydrocarbon group having 11 to 19 carbon atoms, R₂ represents a saturated or unsaturated linear hydrocarbon group having 9 to 19 carbon atoms which may or may not have a hydroxyl group in the 1-position, and the symbol * represents an asymmetric carbon atom, said method comprising the steps of:
- reacting an optically active aminodiol represented by general formula (4): 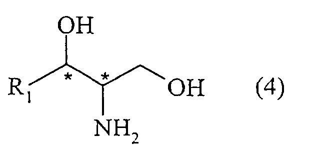
wherein R₁ and the symbol * are the same as R₁ and * in general formula (5), with a fatty acid alkyl ester represented by the general formula (2):
R₂―COO―R₃ (2)
wherein R₂ is the same as R₂ in general formula (5) and R₃ represents a lower alkyl group having 1 to 6 carbon atoms, in a reaction system comprising an alcohol having 3 to 8 carbon atoms and a basic catalyst, said reaction system forming R₃OH as by-product; and
- removing R₃OH, formed during the reaction, from the reaction system;
- recovering the solution from the reaction system and cooling the residue to a temperature comprised between -10°C and room temperature to precipitate crude crystals; and
- collecting and recrystallising the crude crystals in methanol, ethanol or acetonitrile.

3. The method according to claim 2, wherein said optically active primary amide derivative has a (2S, 3R)-configuration.

4. The method according to any one of claims 1 to 3, wherein said alcohol is n-butanol.

5. The method according to any of claims 1 to 4, wherein said removing step comprises carrying out the reaction under a reduced pressure ranging from 3.99 kPa to 13.33 kPa (30 to 100 mm Hg) at a temperature ranging from 90°C to 120°C.

6. The method according to any one of claims 1 to 4, wherein said removing step comprises carrying out the reaction under an inert atmosphere at a temperature ranging from 90°C to 120°C.

7. The method according to any one of claims 1 to 6, wherein the lower alkyl group represented by R₃ is a methyl or an ethyl group.

8. The method according to any one of claims 1 to 7, wherein the fatty acid alkyl ester represented by the general formula (2) is used in an amount ranging from 1 to 1.5 moles per mole of the aminodiol represented by the general formula (1).

9. The method according to any one of claims 1 to 8, wherein the alcohol is used in an amount by weight ranging from 5 to 10 times the amount by weight of the aminodiol represented by the general formula (1).

10. The method according to any one of claims 1 to 9, wherein the basic catalyst is used in an amount ranging from 0.01 to 0.2 mole per mole of the fatty acid alkyl ester represented by the general formula (2).

## Patentansprüche

1. Verfahren zur Herstellung eines primären Amidderivats der allgemeinen Formel (3): 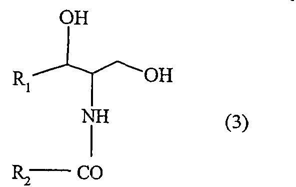 wobei R₁ einen gesättigten linearen Kohlenwasserstoffrest mit 11 bis 19 Kohlenstoffatomen und R₂ einen gesättigten oder ungesättigten linearen Kohlenwasserstoffrest mit 9 bis 19 Kohlenstoffatomen darstellt, der eine Hydroxylgruppe in der 1-Position aufweisen kann oder nicht, umfassend die Schritte:
- Umsetzen eines Aminodiols der allgemeinen Formel (1): 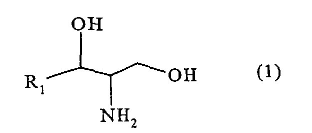 wobei R₁ gleich R₁ der allgemeinen Formel (3) ist, mit einem Fettsäurealkylester der allgemeinen Formel (2):
R₂―COO―R₃ (2)
wobei R₂ gleich R₂ der allgemeinen Formel (3) ist und R₃ einen Niederalkylrest mit 1 bis 6 Kohlenstoffatomen darstellt, in einem Reaktionssystem, das einen Alkohol mit 3 bis 8 Kohlenstoffatomen und einen basischen Katalysator umfaßt, wobei das Reaktionssystem R₃OH als Nebenprodukt bildet; und
- Entfernen von R₃OH, welches während der Reaktion gebildet wird, aus dem Reaktionssystem;
- Wiedergewinnen der Lösung aus dem Reaktionssystem und Abkühlen des Rückstands auf eine Temperatur zwischen -10°C und Raumtemperatur um Rohkristalle auszufällen; und
- Sammeln und Umkristallisieren der Rohkristalle aus Methanol, Ethanol oder Acetonitril.

2. Verfahren zur Herstellung eines optisch aktiven primären Amidderivats der allgemeinen Formel (5): 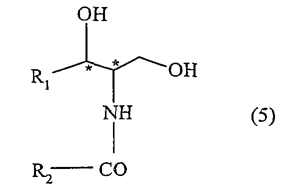 wobei R₁ einen gesättigten linearen Kohlenwasserstoffrest mit 11 bis 19 Kohlenstoffatome und R₂ einen gesättigten oder ungesättigten linearen Kohlenwasserstoffrest mit 9 bis 19 Kohlenstoffatome darstellt, der eine Hydroxylgruppe in der 1-Position aufweisen kann oder nicht, und das Symbol * ein asymmetrisches Kohlenstoffatom darstellt, umfassend die Schritte:
- Umsetzen eines optisch aktiven Aminodiols der allgemeinen Formel (4): 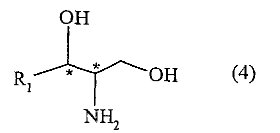 wobei R₁ und das Symbol * gleich R₁ und * der allgemeinen Formel (5) sind, mit einem Fettsäurealkylester der allgemeinen Formel (2):
R₂―COO―R₃ (2)
wobei R₂ gleich R₂ der allgemeinen Formel (5) ist und R₃ einen Niederalkylrest mit 1 bis 6 Kohlenstoffatomen darstellt, in einem Reaktionssystem, das einen Alkohol mit 3 bis 8 Kohlenstoffatomen und einen basischen Katalysator umfaßt, wobei das Reaktionssystem R₃OH als Nebenprodukt bildet; und
- Entfernen von R₃OH, welches während der Reaktion gebildet wird, aus dem Reaktionssystem;
- Wiedergewinnen der Lösung aus dem Reaktionssystem und Abkühlen des Rückstands auf eine Temperatur zwischen -10°C und Raumtemperatur um Rohkristalle auszufällen; und
- Sammeln und Umkristallisieren der Rohkristallc aus Methanol, Ethanol oder Acetonitril.

3. Verfahren nach Anspruch 2, wobei das optisch aktive primare Amidderivat eine (2S, 3R)-Konfiguration aufweist.

4. Verfahren nach einem der Ansprüche 1 bis 3, wobei der Alkohol n-Butanol ist.

5. Verfahren nach einem der Ansprüche 1 bis 4, wobei der Schritt des Entfernens das Ausfühlen der Reaktion unter verringertem Druck im Bereich von 3,99 kPa to 13,33kPa (30 bis 100 mm Hg) bei einer Temperatur im Bereich von 90°C bis 120°C umfaßt.

6. Verfahren nach einem der Ansprüche 1 bis 4, wobei der Schritt des Entfernens das Ausführen der Reaktion in einer Inertatmosphäre bei einer Temperatur im Bereich von 90°C bis 120°C umfaßt.

7. Verfahren nach einem der Ansprüche 1 bis 6, wobei der Niederalkylrest, der durch R₃ dargestellt ist, eine Metyl- oder eine Ethylgruppe ist.

8. Verfahren nach einem der Ansprüche 1 bis 7, wobei der Fettsäurealkylester, der durch die allgemeine Formel (2) dargestellt ist, in einer Menge im Bereich von 1 bis 1,5 Mol pro Mol des Aminodiols, das durch die allgemeine Formel (1) dargestellt ist, verwendet wird.

9. Verfahren nach einem der Ansprüche 1 bis 8, wobei der Alkohol in einer Gewichtsmenge im Bereich der 5 bis 10-fachen Menge des Gewichts des Aminodiols, das durch die allgemeine Formel (1) dargestellt ist, verwendet wird.

10. Verfahren nach einem der Ansprüche 1 bis 9, wobei der basische Katalysator in einer Menge im Bereich von 0,01 bis 0,2 Mol pro Mol des Fettsäurealkylesters, der durch die allgemeine Formel (2) dargestellt ist, verwendet wird.

## Revendications

1. Méthode pour préparer un dérivé d'amide primaire représenté par la formule générale (3): 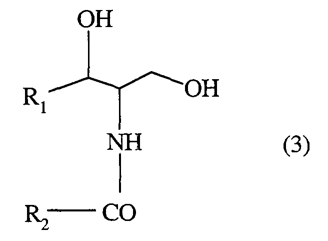 dans laquelle R₁ représente un groupe hydrocarboné linéaire saturé ayant 11 à 19 atomes de carbone, et R₂ représente un groupe hydrocarboné linéaire saturé ou insaturé ayant 9 à 19 atomes de carbone, qui peut avoir ou non un groupe hydroxyle en position 1, ladite méthode comprenant les étapes de :
- réaction d'un aminodiol représenté par la formule générale (1) : 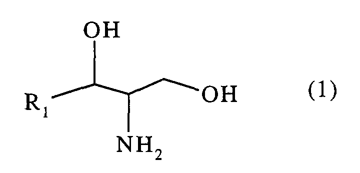 dans laquelle R₁ a la même signification que R₁ dans la formule générale (3), avec un ester alkylique d'un acide gras de formule générale (2) :
R₂―COO―R₃ (2)
dans laquelle R₂ a la même signification que R₂ dans la formule générale (3) et R₃ représente un groupe alkyle inférieur ayant 1 à 6 atomes de carbone, dans un système réactionnel comprenant un alcool ayant 3 à 8 atomes de carbone et un catalyseur basique, ledit système réactionnel formant R₃OH comme sous-produit ; et
- élimination de R₃OH, formé pendant la réaction, du système réactionnel ;
- récupération de la solution à partir du système réactionnel et refroidissement du résidu jusqu'à une température comprise entre -10°C et la température ambiante pour précipiter les cristaux bruts ; et
- collecte et recristallisation des cristaux bruts dans du méthanol, de l'éthanol ou de l'acétonitrile.

2. Méthode pour préparer un dérivé d'amide primaire optiquement actif représenté par la formule générale (5) : 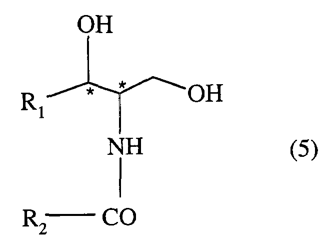 dans laquelle R₁ représente un groupe hydrocarboné linéaire saturé ayant 11 à 19 atomes, R₂ représente un groupe hydrocarboné linéaire saturé ou insaturé ayant 9 à 19 atomes de carbone qui peut avoir ou non un groupe hydroxyle en position 1 et le symbole * représente un carbone asymétrique, ladite méthode comprenant les étapes de :
- réaction d'un aminodiol optiquement actif représenté par la formule générale (4) : 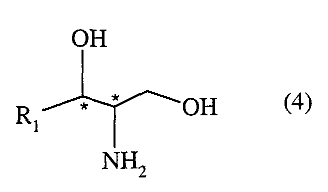 dans laquelle R₁ et le symbole * ont la même signification que R₁ et * dans la formule générale (5), avec un ester alkylique d'acide gras représenté par la formule générale (2) :
R₂―COO―R₃ (2)
dans laquelle R₂ a la même signification que R₂ dans la formule générale (5) et R₃ représente un groupe alkyle inférieur ayant 1 à 6 atomes de carbone, dans un système réactionnel comprenant un alcool ayant 3 à 8 atomes de carbone et un catalyseur basique, ledit système réactionnel formant R₃OH comme sous-produit ; et
- élimination de R₃OH, formé pendant la réaction, du système réactionnel ;
- récupération de la solution à partir du système réactionnel et refroidissement du résidu jusqu'à une température comprise entre -10°C et la température ambiante pour précipiter les cristaux bruts ; et
- collecte et recristallisation des cristaux bruts dans du méthanol, de l'éthanol ou de l'acétonitrile.

3. Méthode selon la revendication 2, dans laquelle ledit dérivé d'amide primaire a la configuration (2S, 3R).

4. Méthode selon l'un quelconque des revendications 1 à 3, dans laquelle ledit alcool est le n-butanol.

5. Méthode selon l'un quelconque des revendications 1 à 4, dans laquelle ladite étape d'élimination comprend la réalisation de la réaction sous une pression réduite allant de 3,99 kPa à 13,33 kPa (30 à 100 mmHg) à une température allant de 90°C à 120 °C.

6. Méthode selon l'un quelconque revendications 1 à 4, dans laquelle ladite étape d'élimination comprend la réalisation de la réaction sous atmosphère inerte à une température allant de 90°C à 120°C.

7. Méthode selon l'un quelconque des revendications 1 à 6, dans laquelle le groupe alkyle inférieur représenté par R₃ est un groupe méthyle ou un groupe éthyle.

8. Méthode selon l'un quelconque des revendications 1 à 7, dans laquelle l'ester alkylique d'acide gras représenté par la formule générale (2) est utilisé à raison de 1 à 1,5 moles par mole d'aminodiol représenté par la formule générale (1).

9. Méthode selon l'un quelconque des revendications 1 à 8, dans laquelle l'alcool est utilisé en une quantité pondérale de 5 à 10 fois la quantité pondérale de l'aminodiol représenté par la formule générale (1).

10. Méthode selon l'un quelconque des revendications 1 à 9, dans laquelle le catalyseur basique est utilisé à raison de 0,01 à 0,2 mole par mole d'ester alkylique d'acide gras représenté par la formule générale (2).
